# EUROPÄISCHE PATENTSCHRIFT

(11) **EP 0 270 482 B1**
(45) Veröffentlichungstag und Bekanntmachung des Hinweises auf die Patenterteilung: **27.10.1993**
(21) Anmeldenummer: 87730149.9
(22) Anmeldetag: 13.11.1987
(51) Int. Cl.: A61K 31/42

(54) **Verwendung von 5-(Subst. Phenyl)- Oxazolidinonderivaten zur Behandlung von Depressionen**
Use of 5-(substituted phenyl-)oxazolidinones in the treatment of depressions
L'emploi des dérivés de l'oxazolidinone 5-(phényl-substituées) pour le traitement des dépressions

(30) Priorität: 14.11.1986 DE 3639225
(43) Veröffentlichungstag der Anmeldung: 08.06.1988
(73) Patentinhaber: SCHERING AKTIENGESELLSCHAFT, 13342 Berlin (DE)
(72) Erfinder: Wachtel, Helmut, Dr., D-1000 Berlin 19 (DE); Schneider, Herbert H., Dr., D-1000 Berlin 15 (DE)

(56) Entgegenhaltungen:
- WO-A-86/02268
- US-A- 4 186 129
- J. MED. CHEM., Band 11, November 1968, Seiten 1121-1125; E.D. BERGMANN et al.: "Some epinephrine analogs"
- J. HETEROCYCLIC CHEM., Band 15, Nr. 3, 1978, Seiten 519-521; R.K. JAISWAL et al.: "Substituted 5-aryl-or 5-diphenylmethyl-3- alkyl-2-oxazolidones as anticonvulsants"
- Allgemeine und spezielle Pharmakologie und Toxikologie, 2. Auflage, 1977, Seiten 447 - 448
- The Merck Manual of Diagnosis and Therapie, 14. Auflage, 1982, Seite 2338

## Beschreibung

Die Erfindung betrifft den Gegenstand der Ansprüche.

Dem Merck Manual of Diagnosis and Therapie ist zu entnehmen, daß MAO-Hemmer als Antidepressiva verwendet werden können und es ist auch bekannt, daß es Verbindungen gibt, die sowohl neuroleptische wie auch antidepressive Wirkung besitzen (Allgemeine und spezielle Pharmakologie und Toxikologie, 2. Auflage, 1977, Seite 447-448).

Aus dem US-Patent 4186129 ist bekannt, daß 5-(Subst. Phenyl)-oxazolidinonderivate phosphodiesterasehemmende Eigenschaften haben und darüberhinaus zentral-depressiv, antidopaminerg, antinoziceptiv und anticonvulsiv wirken. Ferner werden in der WO-A-8602268 weitere 5-(Subst. Phenyl)-oxazolidinone beschrieben, die bei topischer Applikation antiinflammatorische Eigenschaften besitzen. Strukturell ähnliche Oxazolidinone sind aus J. Med. Chem. 11, November 1968, Seiten 1121-1125 und J. Heterocyclic Chem. 15, 1978, Seiten 519-521 bekannt.

Es wurde nun gefunden, daß die Wirkung der oben beschriebenen Verbindungen nicht zentral depressiv sondern zentral antidepressiv ist. 5-(Subst. Phenyl)-oxazolidinone weisen sehr gute neuropsychotrope Wirksamkeit auf.

Erfindungsgemäß gemeint sind insbesondere racemische und optisch aktive 5-(Subst.-phenyl)-oxazolidinone der allgemeinen Formel I 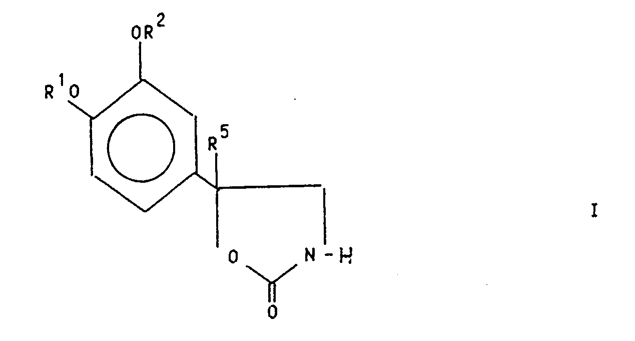
worin
- R¹: C₁₋₄-Alkyl,
- R²: einen C₁₋₈-Kohlenwasserstoffrest oder einen C₃₋₇-Cycloalkylrest, bei dem eine CH₂-Gruppe durch ein Sauerstoffatom ersetzt ist und
- R⁵: Wasserstoff oder C₁₋₄-Alkyl bedeutet .

Die Verbindungen der allgemeinen Formel I besitzen ein asymetrisches Kohlenstoffatom und können daher als Razemat oder nach Auftrennen des Razemats mit den üblichen Methoden als Antipoden verwendet werden.

Die niederen Alkylgruppen enthalten jeweils vorzugsweise 1 - 4 Kohlenstoffatome wie beispielsweise Methyl, Ethyl, Propyl, Isopropyl, Butyl, Isobutyl, tert. Butyl und sek. Butyl.

Die Substituenten R¹ und R² können gleich oder verschieden sein.

Als Kohlenwasserstoffrest R² kommen gesättigte oder ungesättigte, geradkettige oder verzweigte Alkylgruppen mit vorzugsweise 1 - 6 Kohlenwasserstoffatomen in Betracht, ferher Cycloalkyl- und Cycloalkyl-alkylgruppen mit vorzugsweise 3 - 7 Kohlenstoffatomen sowie Aryl- und Aralkylgruppen mit vorzugsweise 6 - 8 Kohlenstoffatomen.

Geeignete gesättigte Alkylgruppen sind die oben genannten niederen Alkylgruppen und noch beispielsweise Pentyl, 2-Methylbutyl, 2,2-Dimethylpropyl und Hexyl.

Als Alkenyl- und Alkinylgruppen seien vorzugsweise genannt:
2-Propenyl, 3-Methyl-2-propenyl, 2-Propinyl,

Bedeutet der Kohlenwasserstoffrest eine Cycloalkylgruppe, so kann eine CH₂-Gruppe des Cycloalkyrestes gegebenenfalls durch ein Sauerstoffatom ersetzt sein. Als cyclischer Ätherrest seien beispielsweise 3-Tetrahydrofuranyl und 3-Tetrahydropyranyl und als Cycloalkylrest beispielsweise Cyclopropyl, Cyclobutyl, Cyclopentyl, Cyclohexyl und Cycloheptyl genannt.

Bedeutet der Kohlenwasserstoffrest eine Cycloalkyl-alkylgruppe, so ist die Cyclopropylmethyl-, Cyclopropylethyl- und Cyclopentylmethylgruppe als bevorzugt anzusehen.

Die Aryl- und Aralkylgruppen R² haben vorzugsweise 6 - 8 Kohlenstoffatome wie beispielsweise Phenyl, Benzyl und Phenethyl.

Insbesondere Verbindungen der allgemeinen Formel I, in denen R¹ eine niedere Alkylgruppe und R² eine C₁₋₆ Alkylgruppe oder einen gegebenenfalls durch ein Sauerstoffatom unterbrochenen Cycloalkylrest darstellt, sind zur Behandlung neuropsychotroper Erkrankungen geeignet, ganz besonders bevorzugt sind Verbindungen der allgemeinen Formel I, in denen R¹ eine Methylgruppe darstellt.

Die Herstellung der Verbindungen der allgemeinen Formel I erfolgt nach den in der WO-A-8602268 und in dem US-Patent 4186129 beschriebenen Verfahren.

Die Wirkung der Verbindungen der allgemeinen Formel I auf das Zentralnervensystem wurde durch Antagonismus der durch Reserpin induzierten Körpertemperatur-Erniedrigung bestimmt.

Die Bestimmung des Antagonismus der durch Reserpin induzierten Erniedrigung der Körpertemperatur erfolgt an männlichen NMRI-Mäusen im Gewicht von 20 - 25 g.

4 Stunden zuvor mit Reserpin (10 mg/kg, i.p.) behandelten Versuchstieren wurden die Prüfsubstanzen in Abhängigkeit von ihrer Wirksamkeit in Dosierungen zwischen 0.00625 und 25 mg/kg i.p. verabreicht und die Rektaltemperatur mit Hilfe eines elektrischen Thermometers (ellab TE3, Thermosonde RM 6) gemessen. Kontrolltiere wurden mit 0.1 ml/10 g Körpergewicht des Lösungsmittels (physiologische NaCl-Lösung mit Zusatz von 100 g/l Cremophor EL^{R}) i.p. behandelt. Die verschiedenen Behandlungsgruppen umfaßten 6 - 8 Mäuse.

Die Signifikanz der Unterschiede zwischen den Mittelwerten der verschiedenen Prüfsubstanz-behandelten Gruppen und der Kontrollgruppe wurden mittels Varianzanalyse in Verbindung mit dem Dunnett-Test ermittelt. Angegeben ist die minimale effektive Dosis (MED), d.h. die niedrigste statistisch signifikant antihypotherm wirksame Dosis.

Die nachfolgende Tabelle zeigt die in diesem Test erhaltenen Ergebnisse.

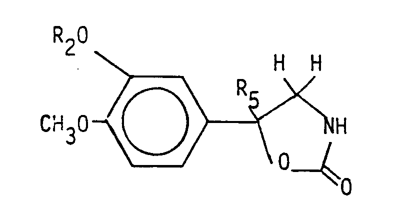

| Nr. | R² | R⁵ | Reserpin-Antag. MED (mg/kg) |
|---|---|---|---|
| 1. | Cyclopentyl | H | 0,39 |
| 2. | Tetrahydrofuranyl | H | 0,1 |
| 3. | C₂H₅ | CH₃ | 0.39 |
| 4. | C₃H₇ | CH₃ | 0.10 |
| 5. | C₄H₉ | CH₃ | 1.56 |
| 6. | Cyclopentyl | CH₃ | 0.39 |

Die Verbindungen der allgemeinen Formel I eignen sich somit zur Herstellung eines Mittels zur symptomatischen Behandlung von Depressionen, das sich durch geringe Nebenwirkungen auszeichnet. Sie können insbesondere verwendet werden bei akuten und chronischen Erkrankungen des depressiven Formenkreises, der endogenen Depression und der Altersdepression.

In der medizinischen Praxis können diese Arzneimittel auf Basis von Oxazolidinonderivaten parenteral wie subcutan, intramuskulär oder intravenös und vor allem per os appliziert werden.

Die tägliche Dosis beträgt 0,1 - 10 mg, vorzugsweise jedoch 0,5 - 5 mg. Die Dosis kann einmalig oder in mehreren Gaben verabfolgt werden. Die erfindungsgemäßen Mittel sind auch für eine Dauerbehandlung geeignet.

Die Herstellung der Arzneimittelspezialitäten erfolgt in an sich bekannter Weise, indem der Wirkstoff mit den in der galenischen Pharmazie gebräuchlichen Trägersubstanzen, Verdünnungsmitteln, Geschmackskorrigentien usw. verarbeitet wird.

Für Injektionen kommen insbesondere wäßrige, aber auch ölige Lösungen sowie Suspensionen infrage.

Zur Herstellung intramuskulärer Depotformen können die Wirkstoffe nach gängigen Methoden in fetten Ölen suspendiert oder gelöst werden. Solche Depotformen enthalten etwa 1 - 10 mg Wirkstoff pro Applikationseinheit; der Wirkstoff wird über eine Zeit von 1 bis 10 Tagen freigegeben.

Die erfindungsgemäßen Arzneimittel sind besonders in Form von Tabletten, Kapseln, Dragees, Pillen, Suspensionen und Lösungen für die orale Applikation geeignet.

Die Wirkstoffmenge pro oraler Applikationseinheit beträgt 0,1 - 5 mg, vorzugsweise 0,1 - 1 mg.

Geeignet sind auch orale Retardformen, die in üblicher Weise, z.B. durch Zugabe von hydrierten Fetten und Verarbeitung mit Harzbildnern und Lacken, erhalten werden.

Tropfen für die orale Applikation können durch wäßrige Lösungen oder Suspensionen des Wirkstoffs in Ölen unter Zugabe von Geschmackskorrigentien und/oder Lösungsvermittlern hergestellt werden. In einer Tagesdosis von 3 x 10 Tropfen können beispielsweise 0,5 - 5 mg enthalten sein.

## Patentansprüche (Patentansprüche für folgende(n) Vertragsstaat(en): AT, BE, CH, DE, FR, GB, IT, LI, LU, NL, SE)

1. Verwendung von razemischen oder optisch aktiven Verbindungen der allgemeinen Formel I 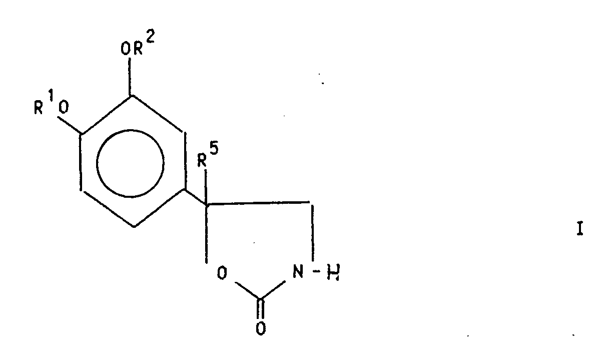 worin
R¹ C₁₋₄-Alkyl,
R² einen C₁₋₈ Kohlenwasserstoffrest oder einen C₃₋₇-Cycloalkylrest, bei dem eine CH₂-Gruppe durch ein Sauerstoffatom ersetzt ist und
R⁵ Wasserstoff oder C₁₋₄-Alkyl bedeutet
zur Herstellung eines Arzneimittels zur Behandlung von Depressionen.

2. Verwendung von Verbindungen der allgemeinen Formel I, worin R¹ Methyl bedeutet, gemäß Anspruch 1.

3. Verwendung von 5-Methyl-5-(3-propoxy-4-methoxyphenyl)-2-oxazolidinon gemäß Patentanspruch 1.

4. Verwendung von 5-(3-Cyclopentyloxy-4-methoxyphenyl)-2-oxazolidinon gemäß Patentanspruch 1.

## Patentansprüche (Patentansprüche für folgende(n) Vertragsstaat(en): GR, ES)

1. Verfahren zur Herstellung eines Arzneimittels zur Behandlung von Depressionen, dadurch gekennzeichnet, daß man razemische oder optisch aktive Verbindungen der Formel I 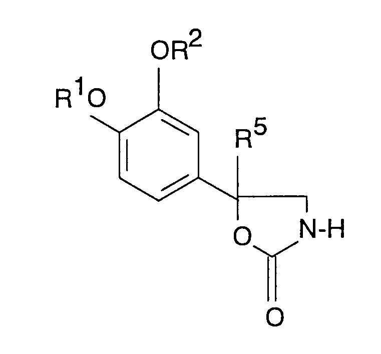 worin
R¹ C₁₋₄-Alkyl,
R² einen C₁₋₈-Kohlenwasserstoffrest oder einen C₃₋₇-Cycloalkylrest, bei dem eine CH₂-Gruppe durch ein Sauerstoffatom ersetzt ist und
R⁵ Wasserstoff oder C₁₋₄-Alkyl bedeutet in einer therapeutisch wirksamen Menge mit inertem Trägermaterial mischt.

2. Verfahren nach Anspruch 1, worin eine Verbindung der Formel I, worin R¹ Methyl bedeutet, verwendet wird.

3. Verfahren nach Anspruch 1, worin 5-Methyl-5-(3-propoxy-4-methoxyphenyl)-2-oxazolidinon verwendet wird.

4. Verfahren nach Anspruch 1, worin 5-(3-Cyclopentyloxy-4-methoxyphenyl)-2-oxazolidinon verwendet wird.

## Claims (Claims for the following Contracting State(s): AT, BE, CH, DE, FR, GB, IT, LI, LU, NL, SE)

1. The use of racemic or optically active compounds of the general formula I 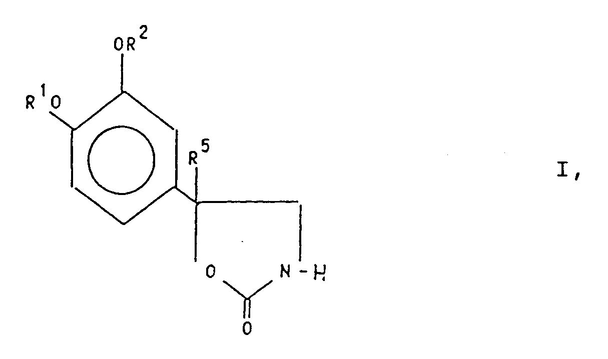 wherein
R¹ represents C₁₋₄-alkyl,
R² represents a C₁₋₈-hydrocarbon radical, or a C₃₋₇-cycloalkyl radical in which one CH₂ group has been replaced by an oxygen atom, and
R⁵ represents hydrogen or C₁₋₄-alkyl,
for the manufacture of a medicament for the treatment of depression.

2. The use of compounds of the general formula I wherein R¹ represents methyl, according to claim 1.

3. The use of 5-methyl-5-(3-propoxy-4-methoxyphenyl)-2-oxazolidinone according to patent claim 1.

4. The use of 5-(3-cyclopentyloxy-4-methoxyphenyl)-2-oxazolidinone according to patent claim 1.

## Claims (Claims for the following Contracting State(s): GR, ES)

1. Process for the manufacture of a medicament for the treatment of depression characterized by an effective amount of racemic or optically active compounds of the general formula I 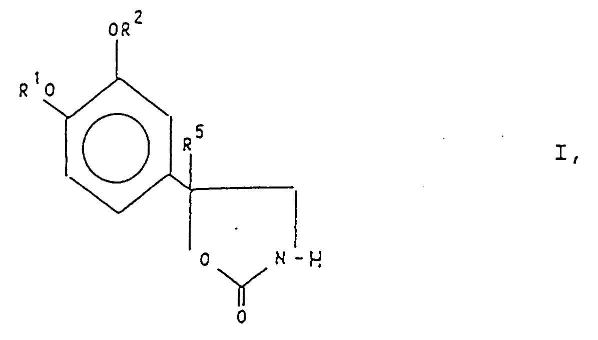 wherein
R¹ represents C₁₋₄-alkyl,
R² represents a C₁₋₈-hydrocarbon radical, or a C₃₋₇-cycloalkyl radical in which one CH₂ group has been replaced by an oxygen atom, and
R⁵ represents hydrogen or C₁₋₄-alkyl, combined with customary expcipients and carriers

2. A process according to claim 1 wherein R¹ represents methyl.

3. A process according to claim 1 wherein 5-methyl-5-(3-propoxy-4-methoxyphenyl)-2-oxazolidinone is used.

4. A process according to claim 1 wherein 5-(3-cyclopentyloxy-4-methoxyphenyl)-2-oxazolidinone is used.

## Revendications (Revendications pour l'(les) Etat(s) contractant(s) suivant(s): AT, BE, CH, DE, FR, GB, IT, LI, LU, NL, SE)

1. Emploi de composés racémiques ou optiquement actifs de formule générale I ci-dessous : 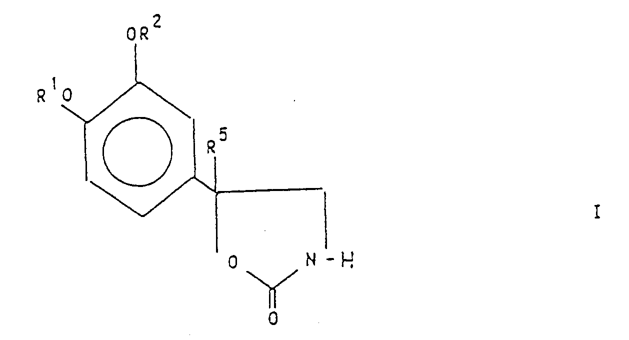 dans laquelle
R¹ représente un alkyle en C₁₋₄,
R² un radical hydrocarboné en C₁₋₈ ou un cycloalkyle en C₃₋₇ dont un groupe CH₂ est remplacé par un atome d'oxygène, et
R⁵ l'hydrogène ou un alkyle en C₁₋₄
pour en préparer un médicament destiné au traitement de dépressions.

2. Emploi de composés de formule I de la revendication 1 dans lesquels R¹ est le groupe méthyle.

3. Emploi de la 5-méthyl-5-(3-propoxy-4-méthoxyphényl)-2-oxazolidinone selon la revendication 1.

4. Emploi de la 5-(3-cyclpentyloxy-4-méthoxyphényl)-2-oxazolidinone selon la revendication 1.

## Revendications (Revendications pour l'(les) Etat(s) contractant(s) suivant(s): GR, ES)

1. Procédé pour en préparer un médicament destiné au traitement de dépressions caracterisé en ce qu'on mélange les composés racémiques ou optiquement actifs de formule générale I ci-dessous: 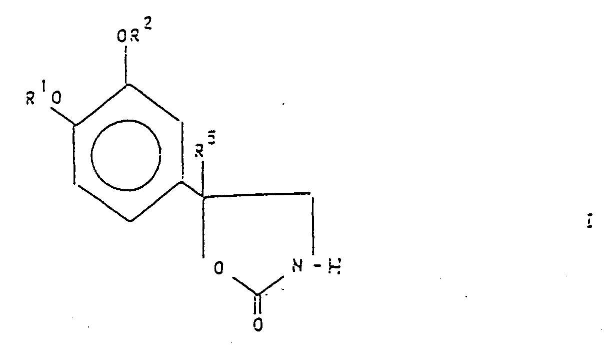 dans laquelle
R¹ représente un alkyle en C₁₋₄,
R² un radical hydrocarboné en C₁₋₈ ou un cycloalkyle en C₃₋₇ dont un groupe CH₂ est remplacé par un atome d'oxygène, et
R⁵ l'hydrogène ou un alkyle en C₁₋₄ avec des substances auxiliaires et des supports classiques.

2. Procédé selon la revendication 1 dans lesquels R¹ est le groupe methyle.

3. Procédé selon la revendication 1dans lequel l'agent thérapeutique est5-methyl-5-(3-propoxy-4-methoxyphenyl)-2-oxazolidinone.

4. Procédé selon la revendication 1 dans lequel l'agent thérapeutique est 5-(3-cyclopentyloxy-4-methoxyphenyl)-2-oxazolidinone.
